# EUROPEAN PATENT APPLICATION

(11) **EP 3 949 936 A1**
(43) Date of publication of application: **09.02.2022**
(21) Application number: 20777587.5
(22) Date of filing: 09.03.2020
(51) Int. Cl.: A61K 6/30, A61K 6/831, A61K 6/882, A61K 6/896

(54) **ADDITION-TYPE SILICONE COMPOSITION FOR DENTAL USE**

(30) Priority: 28.03.2019 JP 2019063512
(71) Applicant: GC Corporation, Sunto-gun, Shizuoka 410-1307 (JP)
(72) Inventor: KAMINOSONO, Yoshiya, Tokyo 174-8585 (JP); OIZUMI, Chiaki, Tokyo 174-8585 (JP); ITO, Teppei, Tokyo 174-8585 (JP)
(74) Representative: Müller-Boré & Partner Patentanwälte PartG mbB
(86) International application number: PCT/JP2020/010030
(87) International publication number: WO 2020/195749

(57) **Abstract**

A dental addtion silicone composition includes methylphenylpolysiloxane, and hydrophobic silica particles, wherein the dental addition silicone composition is substantially free of a surfactant, a BET specific surface area of the hydrophobic silica particles is 30 m²/g or more, and a mass ratio of the methylphenylpolysiloxane to the hydrophobic silica particles is 0.01 to 5.

## Description

### FIELD OF THE INVENTION

The present invention relates to a dental addition silicone composition.

### BACKGROUND OF THE INVENTION

Addition silicone compositions have been widely used in the dental field in the preparation of denture lining materials.

Patent Document 1 discloses a denture temporary relining material composition including five components: (A) 100 parts by weight of an organopolysiloxane containing at least two alkenyl groups in one molecule and having a viscosity of from 0.5 to 20 Pa·s at 25°C; (B) from 0.1 to 40 parts by weight of an organohydrogen polysiloxane containing at least three hydrogen atoms directly bonded to a silicon atom in one molecule; (C) from 10 to 500 ppm, based on the total amount of the components (A) and (B), of a silicone-soluble platinum compound; (D) from 10 to 200 parts by weight of an inorganic filler; (E) from 0.5 to 100 parts by weight of a raw organopolysiloxane rubber containing at least two alkenyl groups in one molecule and having a viscosity of from 1,000 to 20,000 Pa·s at 25°C; and (F) from 1 to 200 parts by weight of a methylphenylpolysiloxane.

### RELATED-ART DOCUMENT

### Patent Documents

Patent Document 1: Japanese Laid-Open Patent Publication No. 2000-245748

### SUMMARY OF THE INVENTION

### Problems to be Solved by the Invention

However, it is desired to further improve the strength of the cured product of the dental addition silicone composition and further suppresses both dripping of the kneaded paste of the dental addition silicone composition and elution of the cured product into water.

In one aspect of the present invention, an object is to provide a dental addition silicone composition capable of improving the strength of the cured product of the dental addition silicone composition and further suppressing both dripping of the kneaded paste of the dental addition silicone composition and elution of the cured product into water.

### Means for Solving the Problems

In one embodiment of the present invention, the present invention provides a dental addition silicone composition including methylphenylpolysiloxane, and hydrophobic silica particles, wherein the dental addition silicone composition is substantially free of a surfactant, wherein a Brunauer-Emmett-Teller (BET) specific surface area of the hydrophobic silica particles is 30 m²/g or more, and wherein a mass ratio of the methylphenylpolysiloxane to the hydrophobic silica particles is 0.01 to 5.

### Effects of the Invention

According to one aspect of the present invention, the invention is to provide a dental addition silicone composition capable of improving the strength of the cured product and also suppressing both dripping of the kneaded paste of the dental addition silicone composition and elution of the cured product into water.

### DETAILED DESCRIPTION OF THE INVENTION

Next, an embodiment for carrying out the present invention will be described.

### [Dental Addition Silicone Composition]

The dental addition silicone composition of the present embodiment contains methylphenylpolysiloxane and hydrophobic silica particles, but is substantially free of a surfactant. Therefore, an elution of a cured product of the dental addition silicone composition into water can be suppressed.

A BET specific surface area of the hydrophobic silica particles is preferably 30 m²/g or more and even more preferably 40 m²/g or more. When the BET specific surface area of the hydrophobic silica particles is less than 30 m²/g, the strength of the cured product of the dental addition silicone composition decreases, and dripping of the kneaded paste of the dental addition silicone composition occurs.

A BET specific surface area of the hydrophobic silica particles is preferably 500 m²/g or less and even more preferably 300 m²/g or less. When the BET specific surface area of the hydrophobic silica particles is 500 m²/g or less, the abrasiveness and cuttability of the cured product of the dental addition silicone composition of the present embodiment are improved.

The mass ratio of methylphenylpolysiloxane to the hydrophobic silica particles in the dental addition silicone composition of the present embodiment is 0.01 to 5 and preferably 0.02 to 3. When the mass ratio of methylphenylpolysiloxane to the hydrophobic silica particles in the dental addition silicone composition is less than 0.01, dripping of a kneaded paste of the dental addition silicone composition occurs. When the mass ratio of methylphenylpolysiloxane to the hydrophobic silica particles in the dental addition silicone composition exceeds 5, the strength of the cured product of the dental addition silicone composition decreases.

The dental addition silicone composition of the present embodiment further preferably contains an organopolysiloxane having two or more alkenyl groups, an organohydrogen polysiloxane, and a hydrosilylation catalyst.

Here, the organopolysiloxane having two or more alkenyl groups and the organohydrogen polysiloxane undergo a hydrosilylation reaction, and thus the dental addition silicone composition can be cured.

### [Organopolysiloxane having two or more of Alkenyl Groups]

An organopolysiloxane having two or more of alkenyl groups is preferably a compound represented by a following average composition formula:

R¹ₐSiO_{(4-a)/2}

wherein R¹ is a substituted or unsubstituted monovalent hydrocarbon group having 1 to 10 carbons, preferably 1 to 8 carbons; a is 1.95 to 2.05, preferably 2.00 to 2.02; and among the a'R¹s, 0.001 to 20% by mol, preferably 0.001 to 10% by mol, more preferably 0.01 to 5% by mol is an alkenyl group having 2 to 8 carbons and preferably 2 to 6 carbons.

Here, the monovalent hydrocarbon group in the R¹ includes, for example, alkyl groups such as methyl group, ethyl group, propyl group, isopropyl group, butyl group, isobutyl group, tert-butyl group, pentyl group, neopentyl group, hexyl group, cyclohexyl group, octyl group, nonyl group, decyl group, and the like; aryl groups such as phenyl group, tolyl group, xylyl group, naphthyl group, and the like; aralkyl groups such as benzyl group, phenylethyl group, phenylpropyl group, and the like; and alkenyl groups such as vinyl group, and allyl group, propenyl group, isopropenyl group, butenyl group, hexenyl group, cyclohexenyl group, octenyl group, and the like.

Examples of substituents in the R¹ include halogen atoms such as fluorine atom, bromine atom, chlorine atom, and the like; cyano group; and the like.

Examples of alkyl groups substituted by substituents include a chloromethyl group, chloropropyl group, bromoethyl group, trifluoropropyl group, cyanoethyl group, and the like.

It should be noted that the alkenyl group in R¹ may be bonded to a silicon atom at the terminal end or may be bonded to a silicon atom at other than the terminal end, but preferably is bonded to a silicon atom at both ends.

In addition, the groups other than the alkenyl group in R¹ is preferably a methyl group or a phenyl group.

Here, the organopolysiloxane having two or more alkenyl groups contains M units (R₃SiO_{1/2}) and D units (R₂SiO), but may further contain T units (RSiO_{3/2}).

In addition, the organopolysiloxane having two or more alkenyl groups can also be either a homopolymer or a copolymer.

Examples of the organopolysiloxane having two or more alkenyl groups include dimethyl polysiloxane having both ends sealed with a dimethylvinyl siloxy groups; dimethyl polysiloxane having both ends sealed with a methyldivinylsiloxy group; a copolymer of dimethylsiloxane (80% by mol) and methylphenylsiloxane (20% by mol) having both ends sealed with a dimethylvinyl siloxy group; a copolymer of dimethylsiloxane (80% by mol) and diphenylsiloxane (20% by mol) having both ends sealed with a dimethylvinyl siloxy group; a copolymer of dimethylsiloxane (90% by mol) and diphenylsiloxane (10% by mol) having both ends sealed with a dimethylvinyl siloxy group; and a copolymer of dimethylsiloxane and methylvinylsiloxane having both ends sealed with a trimethylsiloxy group; and the like.

Two or more kinds of organopolysiloxane having two or more alkenyl groups may be used in combination.

### [Organohydrogenpolysiloxane]

The organohydrogenpolysiloxane is preferably a compound represented by a following average composition formula:

R²_{b}H_{c}SiO_{(4-b-c)/2}

wherein R² is a substituted or unsubstituted monovalent hydrocarbon group of 1 to 10 carbons, b is 0.7 to 2.1, c is 0.001 to 1.0, and b+c is 0.8 to 3.0.

The number of hydrosilyl groups present in the organohydrogenpolysiloxane is preferably 2 to 300, more preferably 3 to 200, and even more preferably 4 to 100.

Here, the R² is similar to the R¹ in the organopolysiloxane having two or more alkenyl groups, but is preferably free from aliphatic unsaturated bond.

Examples of organohydrogenpolysiloxanes include 1,1,3,3-tetramethyldisiloxane, 1,3,5,7-tetramethyl cyclotetrasiloxane, methylhydrogencyclopolysiloxane, methylhydrogensiloxane-dimethylsiloxane cyclic copolymer, tris(dimethylhydrogensiloxy) methylsilane, tris(dimethylhydridrogenesiloxy) phenylsilane, methylhydrogenpolysiloxane having both ends sealed with trimethylsiloxy groups, a copolymer of dimethylsiloxane-methylhydrogensiloxane having both ends sealed with trimethylsiloxy groups, and dimethylpolysiloxane having both ends sealed with dimethylhydrogensiloxy groups, a copolymer of dimethylsiloxane-methylhydrogensiloxane having both ends sealed with dimethylhydrogensiloxy groups, methyl hydrogenpolysiloxane having both ends sealed with dimethylhydrogensiloxy group, a copolymer of methyl hydrogensiloxane-diphenylsiloxane copolymer having both ends sealed with trimethylsiloxy group, a copolymer of methyl hydrogensiloxane-diphenylsiloxane-dimethylsiloxane having both ends sealed with trimethylsiloxy group, a copolymer having (CH₃)₂HSiO_{1/2} units and SiO_{4/2} units, and a copolymer having (CH₃)₂HSiO_{1/2} units, SiO_{4/2} units, (C₆H₅)SiO_{3/2} units, and the like.

The organohydrogenpolysiloxane may be linear, cyclic, or branched.

The number of silicon atoms present in the organohydrogenpolysiloxane is preferably 2 to 1,000, more preferably 3 to 300, and even more preferably 4 to 100.

Two or more kinds of organohydrogenpolysiloxane may be used in combination.

A molar ratio of the hydrosilyl group of the organohydrogenpolysiloxane with respect to the alkenyl group of the organopolysiloxane having two or more alkenyl groups is 0.1 to 4.0.

The mass ratio of organohydrogenpolysiloxane to organopolysiloxane having two or more alkenyl groups is preferably 0.1 to 40%, and even more preferably 0.5 to 30%.

### [Hydrosilylation Catalyst]

The hydrosilylation catalyst is preferably soluble in silicone oil.

Examples of the hydrosilylation catalyst include platinum black, platinum(II) chloride, platinum chloride acid, a reactant of platinum chloride acid and monohydric alcohol, a complex of platinum chloride acid and olefins, a platinum-based catalyst such as platinum bis-acetoacetate, a palladium-based catalyst, a platinum-based metal catalyst such as rhodium-based catalyst, and the like.

In addition, two or more kinds of hydrosilylation catalysts may be used in combination.

The mass ratio of the hydrosilylation catalyst to the total mass of the organopolysiloxane and organohydrogen polysiloxane having two or more alkenyl groups is preferably 0.001 to 0.1%, and even more preferably 0.005 to 0.05%.

### [Methylphenylpolysiloxane]

Methylphenylpolysiloxane is preferably a silicone oil.

The mass ratio of methylphenylpolysiloxane to organopolysiloxane having two or more alkenyl groups is preferably 0.01 to 2, and even more preferably 0.01 to 1.

### [Hydrophobic silica particles]

Hydrophobic silica particles include, for example, silica particles that have been surface treated with a silane coupling agent.

The mass ratio of hydrophobic silica particles to organopolysiloxane having two or more alkenyl groups is preferably 5 to 50% and even more preferably 10 to 45%.

### [Surfactant]

The mass ratio of the surfactant to the organopolysiloxane having two or more alkenyl groups is preferably 3% or less and even more preferably 1% or less.

### [Inorganic Filler]

The dental addition silicone composition of the present embodiment further preferably contains inorganic fillers other than hydrophobic silica particles with a BET specific surface area of 30 m²/g or more (hereafter referred to as inorganic filler). Accordingly, the hardness and cuttability of the cured product of the dental addition silicone composition are improved.

The inorganic filler is not particularly limited to any inorganic filler other than hydrophobic silica particles with a BET specific surface area of 30 m²/g or higher. Examples of the inorganic fillers include aerosol silica particles, wet-type silica particles, crystalline silica particles, carbon black, iron oxide red particles, cerium oxide particles, titanium oxide particles, calcium carbonate particles, aluminum hydroxide particles, titanate particles, and the like.

The median diameter of the inorganic filler is preferably 0.5 to 50 µm and even more preferably 0.5 to 20 µm.

Two or more kinds of inorganic fillers may be used in combination.

The mass ratio of inorganic filler to organopolysiloxane having two or more alkenyl groups is preferably 0.01 to 10 and even more preferably 0.1 to 5.

### [Other Components]

The dental addition silicone composition of the present embodiment may further contain silicone oils other than methylphenylpolysiloxane, raw silicone rubber, and the like.

Silicone oils other than methylphenylpolysiloxane include, for example, dimethylpolysiloxane and the like.

Raw silicone rubber preferably includes a vinyl group.

Raw silicone rubber includes, for example, raw dimethylpolysiloxane rubber, raw methylphenyl rubber, and the like.

### [Method of Using Dental Addition Silicone Composition]

The dental addition silicone composition of the present embodiment is preferably used as a two-component dental addition silicone composition having a first agent containing a hydrosilylation catalyst and a second agent containing an organohydrogen polysiloxane.

As an example, a kneaded paste of a first agent containing a hydrosilylation catalyst and a second agent containing an organohydrogenpolysiloxane is built up in a tray or mouth, and left in a mouth until the kneaded paste hardens.

As another example, a first agent containing a hydrosilylation catalyst and a second agent containing an organohydrogenpolysiloxane are kneaded, molded, and allowed to stand at room temperature or heated to 50 to 200°C.

### [Applications of Dental Addition Silicone Composition]

The dental addition silicone composition of the present embodiment can be applied, for example, to denture lining materials, impression materials, and the like.

### EXAMPLES

Hereinafter, examples of the present invention will be described, but the present invention is not limited to the examples.

### [Examples 1 to 4, Comparative Examples 1 to 4]

In the amounts [parts by mass] indicated in Table 1, vinyl-terminated dimethylpolysiloxane, methylhydrogenpolysiloxane, silica particles, hydrosilylation catalyst, hydrophobic silica particles, methylphenylpolysiloxane, dimethylpolysiloxane, raw dimethylpolysiloxane rubber, and polyoxyethylene alkyl ether were mixed to prepare a two-component dental addition silicone composition with paste A (Catalyst paste) and paste B (Base paste).

The details of each component in Table 1 are as follows.

Vinyl-terminated dimethylpolysiloxane A: dimethylpolysiloxane having a viscosity of 30 Pa·s at 25°C and both ends are sealed with a vinyldimethylsiloxy groups.

Vinyl-terminated dimethylpolysiloxane B: dimethylpolysiloxane having a viscosity of 3 Pa·s at 25°C and both ends are sealed with a vinyldimethylsiloxy group.

Vinyl-terminated dimethylpolysiloxane C: dimethylpolysiloxane having a viscosity of 20 Pa·s at 25°C and both ends are sealed with a vinyldimethylsiloxy group.

Vinyl-terminated dimethylpolysiloxane D: dimethylpolysiloxane having a viscosity of 100 Pa·s at 25°C and both ends are sealed with a vinyldimethylsiloxy group.

Methylhydrogenpolysiloxane: a linear methylhydrogenpolysiloxane containing 40% by mol of methylhydrogensiloxy groups.

Silica particles A: fused quartz particles with a median diameter of 6 µm Fuselex E2 (manufactured by Tatsumori Ltd.).

Silica particles B: fused quartz particles with a median diameter of 10 µm Fuselex E1 (manufactured by Tatsumori Ltd.).

Silica particles C: fused quartz particles with a median diameter of 1.5 µm Fuselex WX (manufactured by Tatsumori Ltd.).

Hydrosilylation catalyst: 0.4% by mass silicone oil solution of platinum 1,3-divinyl-1,1,3,3-tetramethyldisiloxane complex.

Hydrophobic silica particles A: hydrophobic fumed silica AEROSIL RX200 (manufactured by Aerosil Japan) with a BET specific surface area of 140 m²/g.

Hydrophobic silica particles B: hydrophobic fumed silica AEROSIL RX50 (manufactured by Aerosil Japan) with a BET specific surface area of 35 m²/g.

Hydrophobic silica particles C: hydrophobic fumed silica AEROSIL RX300 (manufactured by Aerosil Japan) with a BET specific surface area of 210 m²/g.

Methylphenylpolysiloxane A: silicone oil KF50-100cs (manufactured by Shin-Etsu Silicone) with a kinematic viscosity of 100 mm²/s at 25°C.

Methylphenylpolysiloxane B: silicone oil KF50-1000cs (manufactured by Shinetsu Silicone) with a kinematic viscosity of 1000 mm²/s at 25°C.

Dimethylpolysiloxane: silicone oil KF96-100cs (manufactured by Shin-Etsu Silicone) with a kinematic viscosity of 100 mm²/s at 25°C.

Raw dimethylpolysiloxane rubber: raw dimethylpolysiloxane rubber having a viscosity of 5000 Pa·s at 25°C and an amount of vinyl group is 0.07% by mol.

Polyoxyethylene alkyl ether: surfactant Naroacty CL40 (manufactured by Sanyo Chemical Industries, Ltd.).

In addition, the mass ratio in Table 1 refers to the mass ratio of methylphenylpolysiloxane to hydrophobic silica particles.

### [Median Diameter of Silica Particles]

The median diameter of the silica particles was measured using a laser diffraction/scattering particle size analyzer LA-950 (manufactured by Horiba).

### [BET specific surface area of hydrophobic silica particles]

The BET specific surface area of hydrophobic silica particles was measured by DIN 66131 (volumetric method).

Next, the tear strength of the cured product of the two-component dental addition silicone composition, dripping of the kneaded paste, and elution of the cured product into water were evaluated.

### [Tear Strength of Cured Product]

After paste A and paste B were kneaded in a mass ratio of 1:1, the kneaded paste was injected into an uncut angle mold in accordance with JIS K 6252, and cured in water at 37°C for 5 minutes. Next, the cured product was removed from the mold and immersed in water at 37°C for one day, upon which a tearing test was conducted.0

### [Dripping of Kneaded Paste]

After paste A and paste B were kneaded at a mass ratio of 1:1, the kneaded paste was placed on the edge of a model of a denture base, it was checked whether the paste dripped from the edge, and then dripping of the kneaded paste was evaluated.

### [Elution of Cured Product into water]

After paste A and paste B were kneaded at a mass ratio of 1:1, the kneaded paste was injected into a metal ring with an inner diameter of 20 mm and a height of 8 mm, and allowed to cure for 30 minutes at room temperature. Next, after the cured product was removed from the mold, the cured product was immersed in water at 37°C. The elution of the cured product into water was evaluated by checking whether eluate was visible or not.

Table 1 indicates the evaluation results of tear strength of the cured product of the two-component dental addition silicone composition, dripping of the kneaded paste, and the elution of the cured product into water.

**[Table 1]**

| | Examples | | | | | | | | Comparative Examples | | | | | | | |
|---|---|---|---|---|---|---|---|---|---|---|---|---|---|---|---|---|
| | 1 | | 2 | | 3 | | 4 | | 1 | | 2 | | 3 | | 4 | |
| Paste | A | B | A | B | A | B | A | B | A | B | A | B | A | B | A | B |
| Vinyl-terminated dimethylpolysiloxane A | 100 | 100 | | | | | | | 100 | 100 | 100 | 100 | | | | |
| Vinyl-terminated dimethylpolysiloxane B | | | 100 | 100 | | | | | | | | | | | | |
| Vinyl-terminated dimethylpolysiloxane C | | | | | 100 | 100 | | | | | | | 100 | 100 | 100 | 100 |
| Vinyl-terminated dimethylpolysiloxane D | | | | | | | 100 | 100 | | | | | | | | |
| Methylhydrogenpolysiloxane | | 3 | | 3 | | 3 | | 3 | | 3 | | 3 | | 3 | | 3 |
| Silica particles A | 30 | 30 | | | | | 20 | 20 | 30 | 30 | 30 | 30 | | | | |
| Silica particles B | | | 500 | 500 | | | | | | | | | | | | |
| Silica particles C | | | | | | | | | | | | | 5 | 5 | 5 | 5 |
| Hydrosilylation catalyst | 3 | | 3 | | 3 | | 3 | | 3 | | 3 | | 3 | | 3 | |
| Hydrophobic silica particles A | 20 | 20 | | | | | 20 | 20 | | | 20 | 20 | | | | |
| Hydrophobic silica particles B | | | 50 | 50 | | | | | | | | | | | | |
| Hydrophobic silica particles C | | | | | 10 | 10 | | | | | | | 10 | 10 | 10 | 10 |
| Methylphenylpolysiloxane A | 5 | 5 | | | 15 | 15 | 5 | 5 | 5 | 5 | | | 10 | 10 | 200 | 200 |
| Methylphenylpolysiloxane B | | | 0.5 | 0.5 | | | | | | | | | | | | |
| Dimethylpolysiloxane | | | | | | | 5 | 5 | | | | | | | | |
| Dimethylpolysiloxane raw rubber | | | | | | | 1 | 1 | | | | | | | | |
| Polyoxyethylene alkyl ether | | | | | | | | | | | | | | 10 | | |
| Mass raio | 0.25 | | 0.01 | | 1.5 | | 0.25 | | | | 0 | | 1 | | 20 | |
| Tear strength [N/mm] | 9.7 | | 11.2 | | 8.8 | | 9.5 | | 3.2 | | 9.5 | | 7.5 | | 4.7 | |
| Dripping | Not observed | | Not observed | | Not observed | | Not observed | | Observed | | Observed | | Not observed | | Not observed | |
| Elution into water | Not observed | | Not observed | | Not observed | | Not observed | | Not observed | | Not observed | | Observed | | Not observed | |

From Table 1, it can be seen that the two-component dental addition silicone compositions of Examples 1 to 4 have high tear strength of the cured product, and are capable of suppressing both dripping of the kneaded paste and elution of the cured product into water.

In contrast, the two-component dental addition silicone composition of Comparative Example 1 does not contain hydrophobic silica particles, and as a result the tear strength of the cured product is low and dripping of the kneaded paste occurs.

The two-component dental addition silicone composition of Comparative Example 2 does not contain methylphenylpolysiloxane, and as a result dripping of the kneaded paste occurs.

The two-component dental addition silicone composition of Comparative Example 3 contains a surfactant, and as a result elution of the cured product into water occurs.

The two-component dental addition silicone composition of Comparative Example 4 has a low tear strength of the cured product because the mass ratio of methylphenylpolysiloxane to hydrophobic silica particles is 20.

This application is based on and claims priority to Japanese patent application No. 2019-063512, filed March 28, 2019 with the Japan Patent Office, the entirety of which is incorporated herein by reference.

## Claims

1. A dental addition silicone composition comprising:
methylphenylpolysiloxane; and
hydrophobic silica particles, wherein
the dental addition silicone composition is substantially free of a surfactant,
a BET specific surface area of the hydrophobic silica particles is 30 m²/g or more, and
a mass ratio of the methylphenylpolysiloxane to the hydrophobic silica particles is 0.01 to 5.

2. The dental addition silicone composition according to claim 1, further comprising an inorganic filler.

3. The dental addition silicone composition according to claim 1, wherein the composition is used for production of denture lining materials.
